# EUROPEAN PATENT SPECIFICATION

(11) **EP 3 106 158 B1**
(45) Date of publication and mention of the grant of the patent: **25.08.2021**
(21) Application number: 16175089.8
(22) Date of filing: 17.06.2016
(51) Int. Cl.: A61K 31/122, A61P 27/02

(54) **VITAMIN K2 AND MACULA DEGENERATION**
VITAMIN K2 UND MAKULADEGENERATION
VITAMINE K2 ET LA DÉGÉNÉRESCENCE MACULAIRE

(30) Priority: 19.06.2015 NL 2015000
(43) Date of publication of application: 21.12.2016
(73) Proprietor: Wagenaar, Louis Johan, 2314 GC Leiden (NL)
(72) Inventor: WAGENAAR, Louis Johan, 2314 GC LEIDEN (NL)
(74) Representative: EP&C

(56) References cited:
- WO-A1-2009/100271
- Anonymous: "The Power of Natto - by Auntyby Aunty", , 13 March 2013 (2013-03-13), XP055300823, Retrieved from the Internet: URL:http://honoluluaunty.com/the-power-of- natto/ [retrieved on 2016-09-08]
- U GRÖBER ET AL: "Vitamin K: an old vitamin in a new perspective", DERMATO-ENDOCRINOLOGY, vol. 6, no. 1, 1 January 2014 (2014-01-01) , page e968490, XP055300524, DOI: 10.4161/19381972.2014.968490
- Rohini Vishwanathan ET AL: "A Systematic Review on Zinc for the Prevention and Treatment of Age-Related Macular Degeneration", Investigative Opthalmology & Visual Science, vol. 54, no. 6, 12 June 2013 (2013-06-12), pages 3985-3998, XP055497677, US ISSN: 1552-5783, DOI: 10.1167/iovs.12-11552

## Description

It has been known for years that macula degeneration is a main cause for serious loss of sight and the occurrence of blindness of the elderly. However, so far, no proven effective treatment to prevent this serious disease has been found. Neither expensive and unpleasant treatments like injections straight into the eye nor the application of cell growth inhibiting substances nor the use of antioxidants (as currently advised by ophthalmologists) have delivered clear preventative or effective treatments until now.

The present invention aims to come to a more effective way to prevent or treat macula degeneration as diagnosed by ophthalmologists and in the case of treatment it will preferentially cease any further worsening or even, whenever possible, achieve improvement in vision.

This objective is reached by administering vitamin K2 in combination with zinc, and wherein the zinc is in a dosage of 2-75mg.

Vitamin K2 - menachinon, menaquinon, or in short MK - has side chains based on repeating unsaturated 5 carbon prenyl units. The length of the side-chains varies and the number of prenyl units is indicated with the number after the MK-abbreviation. For example, MK-4 - menachinon 4. The MK 4 form can (also) be produced by man and animal; it can be found in low concentrations in e.g. meat and eggs. MK-6, MK-7, MK-8 and MK-9 and other MK-chains larger than MK-9 are forms that are produced by bacteria and therefore can be found in fermented food such as for instance Dutch solid cheeses or in animals that feed on predigested food like eels and flatfish. Especially natto, a Japanese food, is a rich source of MK-7. Also in the microbial flora of the large intestine a limited production of vitamin K2 can be found. Whether or not this Vitamin K is able to be absorbed well is still part of debate. Fat soluble vitamins are mainly absorbed in the ileum . The various forms of vitamin K2 mentioned here are all applicable for the present invention.

U GROBER ET AL: "Vitamin K: an old vitamin in a new perspective",DERMATO-ENDOCRINOLOGY, vol. 6, no. 1, 1 January 2014 (2014-01-01),is a review article about Vitamin K; among the therapeutic indications it is stated that vitamin K might be of interest in treating age related macular degeneration, which studies will need to prove in the coming years.

At the moment it seems that variations of vitamin K2 that have a side chain of 7 to 9 carbon atoms are the most effective and already are active at a low dose. But also forms of C-4 are possible. A dose starting from micrograms to many milligrams will be effective. Because vitamin K2 is not harmful, much higher doses can be used such as a gram-range. It is possible that a dose in the range of milligrams or even grams will be more effective than micrograms, but preference is given to the range of 50-500 microgram per day. It is also possible that a dose is given once every 2 days or even less frequently. However, a daily dose will be more effective.

Vitamin K2 can also successfully be combined with other substances like zinc (salts), particularly when the user has shortages thereof (clinically or analytically) and certainly in the case of patients with macula degeneration. Such combinations can be given in separate forms to a patient, for instance vitamin K2 plus zinc in a separate capsule/tablet.

can be used in various forms. For example as an organic or anorganic compound. Organic Zinc compounds that can be used are : L-aspartate, -acetate, -arginate, -Ascorbate, -caprylate, -citrate, -cysteinate, - fumarate, - gluconate, -glutamate, -(bis-)glycinate, -ketoglutarate, -lactate, lysinate, -malate, - methionione, -nicotinate, -orotate,-salicylate,-succinate, -tartrate, -taurinate, Zincgluconate is preferred for Zinc.

Examples of inorganic forms of Zinc are oxides, hydroxides, suplhates and chloride. An example of a daily dosage contains 2-75 mg Zinc.

Vitamin K2 can be applied in all desired forms,

No application form is excluded for applying vitamin K.

### Examples

### Patient 1 - not within scope of claims

A 96-year old woman has suffered for years from macula degeneration. During periodic check-ups (around every 5-6 months) her ophthalmologist has established that her sight is steadily deteriorating. After an intervention consisting of daily oral intake of oily drops containing a total of 360 micrograms of Vitamin K2 and 80 micrograms of vitamin D3 the ophthalmologist established that the macula degeneration has stabilised.

### Patient 2

A 76-year old man has suffered from macula degeneration for a number of years. One of his eyes has a sight of only 5% (according to the current definition). This has been determined at regular check-ups by his ophthalmologist..After an intervention existing of a daily dose of 360 micrograms of vitamin K2 and 80 micrograms of vitamin D3 given in the form of oil droplets and by taking 105 mg of pure Magnesium in the form of three Mg-lactate 350 mg tablets plus 10 milligrams of Zinc in the form of Zinc gluconate, the following was observed. At the next check-up his ophthalmologist was surprised that his sight in the other ('good') eye - which previously had a sight of only 40 % at his previous check up - had even improved to 50 %, while the 'bad' eye had not worsened. Although this patient had to stop for a while with vitamin K2 because of a temporarily use of Marcoumar, he was able to keep the macula degeneration stable after picking up with a further treatment of 180 micrograms of Vitamin K2 and 40 mcg Vitamin D3 plus 70 mg of pure Magnesium in the form of Magnesiumlactate tablets plus 10 micrograms of Zinc in the form of Zinc gluconate.

### Patient 3

A 71 year-old man also suffered from macula degeneration. Periodic visits to an ophthalmologist confirmed that both eyes were affected and progressively deteriorating. After intervention with a daily oral intake of 360 micrograms of vitamin K2 plus 80 micrograms of vitamin D3 plus 175 mg of pure Magnesium, given as Magnesium lactate and 20 microgram Zinc as methionine, the macula degeneration did not progress any further, but instead some improvement in his sight in both eyes could be determined by his ophthalmologist. After half a year of treatment the patient cut his doses of Vitamin D3 and K2 in half. The macula degeneration stabilised at the level reached with half of the original treatment with doses of vitamin K and D plus the original doses of Zinc and Magnesium..

### Patient 4

Over the years a 86-year old woman had experienced continually increasing (wet) macula degeneration. Although her ophthalmologist injected her eyes regularly over the years with a VEGF inhibitor her macula degeneration continued to worsen. However, by taking a daily dose of 360 micrograms of vitamin K2 plus 80 mcg grams of vitamin D3 combined with 5 tablets of 350 mg Magnesium lactate (containing a total of 175 mg pure Magnesium) plus one tablet containing 12 milligrams of Zinc as gluconate plus 2 tablets containing 5 mg Zinc as gluconate each divided over the day, she arrived at a complete drying out of the wet form of her macula degeneration within 2 months. Her (eye-)situation has stabilised now.

## Claims

1. Vitamin K2 for use in the prevention or treatment of macula degeneration, wherein the vitamin K2 is in combination with zinc, and wherein the zinc is in a dosage of 2-75 mg.

2. Vitamin K2 for use according to claim 1, wherein vitamin K2 is dissolved in an oil.

3. Vitamin K2 for use according to any one of the previous claims, wherein the use is by means of oral administration.

4. Vitamin K2 for use according to one of the preceding claims wherein the use of vitamin K2 is daily administration of a dose of between 1 microgram to 10 gram.

5. Vitamin K2 for use according to one of the preceding claims wherein vitamin K2 is combined with magnesium for use in the prevention or treatment of macula degeneration.

6. Vitamin K2 for use according to one of the preceding claims wherein vitamin K2 is combined with vitamin D for use in the prevention or treatment of macula degeneration.

## Patentansprüche

1. Vitamin K2 zur Anwendung bei der Prävention oder der Behandlung von Makuladegeneration, wobei das Vitamin K2 in Kombination mit Zink ist, und worin das Zink in einer Dosierung von 2-75 mg vorliegt.

2. Vitamin K2 zur Anwendung gemäß Anspruch 1, wobei Vitamin K2 in Öl gelöst ist.

3. Vitamin K2 zur Anwendung gemäß nach einem der vorangehenden Ansprüche, wobei die Anwendung mittels oraler Verabreichung erfolgt.

4. Vitamin K2 zur Anwendung gemäß nach einem der vorangehenden Ansprüche, wobei die Anwendung von Vitamin K2 die tägliche Verabreichung einer Dosis zwischen 1 Mikrogramm bis 10 Gramm ist.

5. Vitamin K2 zur Anwendung gemäß nach einem der vorangehenden Ansprüche, wobei Vitamin K2 mit Magnesium zur Anwendung bei der Prävention oder Behandlung von Makuladegeneration kombiniert ist.

6. Vitamin K2 zur Anwendung gemäß nach einem der vorangehenden Ansprüche, wobei Vitamin K2 mit Vitamin D zur Anwendung bei der Prävention oder Behandlung von Makuladegeneration kombiniert ist.

## Revendications

1. Vitamine K2 pour une utilisation dans la prévention ou le traitement de la dégénérescence de la macula, dans laquelle la vitamine K2 est en combinaison avec du zinc, et dans laquelle le zinc est dans un dosage de 2 à 75 mg.

2. Vitamine K2 pour une utilisation selon la revendication 1, dans laquelle la vitamine K2 est dissoute dans une huile.

3. Vitamine K2 pour une utilisation selon l'une quelconque des revendications précédentes, dans laquelle l'utilisation se fait par voie orale.

4. Vitamine K2 pour une utilisation selon l'une des revendications précédentes, dans laquelle l'utilisation de la vitamine K2 est une administration quotidienne d'une dose comprise entre 1 microgramme et 10 grammes.

5. Vitamine K2 pour une utilisation selon l'une des revendications précédentes dans laquelle la vitamine K2 est combinée avec du magnésium pour une utilisation dans la prévention ou le traitement de la dégénérescence de la macula.

6. Vitamine K2 pour une utilisation selon l'une des revendications précédentes dans laquelle la vitamine K2 est combinée avec de la vitamine D pour une utilisation dans la prévention ou le traitement de la dégénérescence de la macula.
